# EUROPEAN PATENT SPECIFICATION

(11) **EP 2 332 655 B1**
(45) Date of publication and mention of the grant of the patent: **27.03.2013**
(21) Application number: 10014936.8
(22) Date of filing: 24.11.2010
(51) Int. Cl.: B01L 9/00, B65D 1/24, A61B 5/145, B65D 25/10

(54) **Test strip container with expandable insert and methods of manufacturing and utilization thereof**
Teststreifenbehälter mit ausdehnbarem Einsatz und Verfahren zur Herstellung und Verwendung davon
Conteneur de bandelette réactive avec insert extensible et procédés de fabrication et d'utilisation associés

(30) Priority: 30.11.2009 US 627358
(43) Date of publication of application: 15.06.2011
(73) Proprietor: Roche Diagnostics GmbH, 68305 Mannheim (DE); F.Hoffmann-La Roche AG, 4070 Basel (CH)
(72) Inventor: Chan, Frank A., Sunnyvale, CA 94087 (US); Groll, Henning, Tucson, AZ 85737 (US)

(56) References cited:
- WO-A1-96/33108
- WO-A1-03/005909
- WO-A2-02/059600
- WO-A2-2009/140627
- GB-A- 2 390 602
- US-A- 4 911 344
- US-A1- 2002 179 609

## Description

### Technical Field

Embodiments of the present disclosure relate generally to containers for test strips, and especially to a test strip container with expandable insert, and methods of manufacturing and utilization thereof.

### Background

Apparatuses and methods for testing compositions of biological fluids, as well as test strips for use in such devices are well known. Typically, test strips are stored in a separate disposable vial, distinct from the test apparatus that analyzes the fluid sample. A test strip is first removed from the vial container, a sample of biological fluid is deposited onto the strip, and the strip is inserted into a test strip meter for analysis of the desired component. After the analysis is complete, the test strip is ejected from the meter, and disposed of.

A problem with test strips is that they are sensitive to environmental degradation due to air and water exposure. Prior art attempts to slow environmental degradation have proven only marginally effective. For example, in one conventional vial a lip seal is provided, in which the displacement of test strips can push them through the seal, and out of the container. In such an arrangement the vial remains sealed except when a strip is removed through the seal. This is ineffective to prevent environmental degradation due to the large amount of air and water ingress during strip removal.

Attempts have been made to protect the strips in a container by adding a desiccant to the chamber to absorb any moisture that enters the chamber during strip removal. However, these attempts have been ineffective because of the large amount of air ingress that occurs through repeated opening and closing of the container.

Test strips may also be packaged individually in tear-away packages, which ensures that the individual strips do not suffer environmental degradation. For instance, blister-type packaging methods may be used. In this configuration, the packages could include desiccant material to maintain the proper humidity in the package. In order for a person to use a single test strip, the package must be opened by tearing the seal. Opening of these packages can be difficult, especially for one with impaired circulation. Furthermore, carrying enough blister packs for a proper testing routine may be inconvenient, and cumbersome.

Test devices are known in which a plurality of test strips are provided on a cartridge disc, with each strip housed in a separate slot. A means is then provided to eject a test strip from its slot when required. U.S. Pat. No. 4,911,344 to Kahler discloses a strip dispenser box capable of dispensing a single test strip from a stack of test strips that does not require the user to insert a finger inside a vessel to retrieve a test strip. Rather, the '344 patent discloses a cap assembly with a strip feeder mechanism mounted to a housing having a magazine capable of holding a stack of test strips. The cap has a slot therein and a slide bar assembly slideably mounted in the slot for moving a test strip out of the dispenser, more particularly out of a gasket-sealed opening positioned on the cap assembly. However, the device disclosed in the '344 patent suffers from certain disadvantages. First and foremost, the strip dispenser of the '344 patent fails to maintain a completely moisture free environment. Specifically, at least two areas of the dispenser permit moisture to enter the housing and thus contact the test strips therein.

The first area which fails to provide a moisture free seal is the slot/slide bar assembly area. The slide bar is made of a cross shaped base member slideably positioned on the inner surface of the cap body and a finger grip which extends upward through the slot of the cap assembly. In operation, the finger grip is driven forward by the action of the thumb of the user and it carries a test strip out an opening of the cap assembly. As described in the patent, the preferred material of the dispenser is polyethylene plastic. In other words, both the cap assembly (the slot area) and the slide bar are made of polyethylene plastic. It will be apparent to one of skill in the art that such an assembly of two contacting polyethylene plastic surfaces cannot provide an adequate barrier to moisture.

The second area which fails to provide a moisture free seal is the opening through which a test strip is removed from the dispenser, even though a seal strip or gasket extends into the dispenser and covers the opening thereto. In other words, a gasket or the like is attached on a first side to the cap assembly and unattached on its other sides to allow a test strip to be pushed therethrough. It will similarly be apparent to one of skill in the art that such a seal cannot provide a barrier to moisture.

A problem with these test strip dispensers is the large size, and lack of portability which makes regular testing inconvenient. Furthermore, it is inconvenient for test strip users to carry around several distinct devices in order to perform routine testing.

Conventional storage vials are small, cylindrical containers that make it difficult to extract a single strip without spilling the entire contents of the vial. A user must invert the storage vial to extract a strip. Unfortunately, during inversion, multiple strips exit the vial, and a user must retrieve those strips from the ground. Additionally, the storage vial does not provide an adequate environmental barrier to prevent the degradation of the test strips.

WO 2009/140627 A2 discloses a container with a compressible insert and a lid. The compressible insert comprises a sealing gasket. When the lid is closed the sealing gasket is compressed and the container is sealed against moisture.

There exists a need for a small portable container that provides test strips to users. The container must be easily used by the user. Furthermore, the container must provide improved environmental protection to the test strips stored therein.

### Summary

It is against the above background that embodiments of the present disclosure provide a test strip container with an expandable insert, and methods of manufacturing and utilization thereof according to the appended claims.

In one embodiment, a container for storing a plurality of test strips is proveded with each test strip having a reagent portion and a handling portion. The container comprises a housing defining a cavity, a lid hingedly connected to the housing to close the cavity, and a compressible insert removably mounted in the cavity.

The insert further comprises ridges which operate to form a barrier against air and water ingress into the cavity, whereby said ridges comprise a raised portion of said compressible insert.

The compressible insert is expandable to retain the plurality of test strips in the cavity. The compressible insert may be folded to encompass a reagent portion of the plurality of test strips. The compressible insert may further comprise a plurality of cooperating ridges. The compressible insert may further comprise a coating.

Still another embodiment, is a method for storing a plurality of test strips according to claim 10.

Yet another embodiment is a method of manufacturing a container for storing a plurality of test strips according to claim 13. The method comprises providing a housing with a defined cavity and a lid hingedly connected to the housing for closing the cavity. A compressible insert is folded and inserted into the cavity. The compressible insert is expandable to retain the plurality of test strips in the cavity.

A method of protecting test strips from environmental degradation may comprise utilizing a container according to an embodiment of the present disclosure.

These and other features and advantageous of these and other various embodiments according to the present disclosure will become more apparent in view the drawings, detailed description, and claims provided that follow hereafter.

### Brief Description of the Drawings

The following detailed description of the embodiments of the present disclosure can be best understood when read in conjunction with the following drawings, where like structure is indicated with like reference numerals, and in which:

FIG. 1 shows a perspective view of a test strip container with a housing, a lid, and a cavity within the housing.

FIG. 2 shows a perspective view of test strip with a handling portion and a reagent portion.

FIG. 3A shows a perspective view of a test strip container with a housing, and a lid.

FIGS. 3B-D show a cross sectional view of a test strip container with a housing and a lid.

FIG. 4 shows a perspective view of a compressible insert in combination with a test strip container.

FIG. 5 shows a cross-sectional view of a compressible insert in a housing storing a test strip with a series of ridges in accordance with one embodiment.

FIG. 6 shows a perspective view of a compressible insert with a slot in combination with a storage container .

FIG. 7 shows a perspective view of a compressible insert with a series of slots.

FIG. 8 shows a perspective view of a partially folded insert with a series of cooperating ridges encompassing a plurality of test strips in combination with a test strip container in accordance with another embodiment.

FIG. 9 shows a perspective view of a folded insert placed within a cavity of a test strip container.

FIG. 10 shows a cross-sectional view of a test strip container in combination with a plurality of test strips in accordance with another embodiment.

FIG. 11 shows a perspective view of a test strip container in combination with a test strip meter.

Skilled artisans appreciate that elements in the figures are illustrated for simplicity and clarity and have not necessarily been drawn to scale. For example, the dimensions of some of the elements in the figures may be exaggerated relative to other elements, as well as conventional parts removed, to help to improve understanding of the various embodiments of the present disclosure.

### Detailed Description

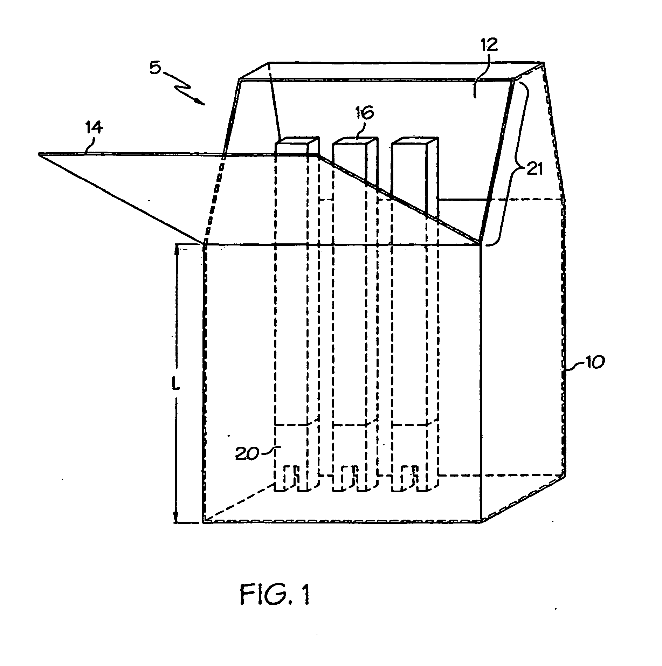

With reference to FIG. 1, a test strip container 5 is disclosed for storing and dispensing test strips 16, and particularly for easily dispensing a single test strip 16from a plurality of test strips 16, i.e., dispensing one test strip 16at a time. Additionally, the container 5 protects retained test strips 16 from adverse contaminants and conditions such as air, light, humidity, dust, dirt, and oils, or other contaminants. The container 5 also allows for easy re-loading of additional test strips 16, as will be apparent from the descriptions below.

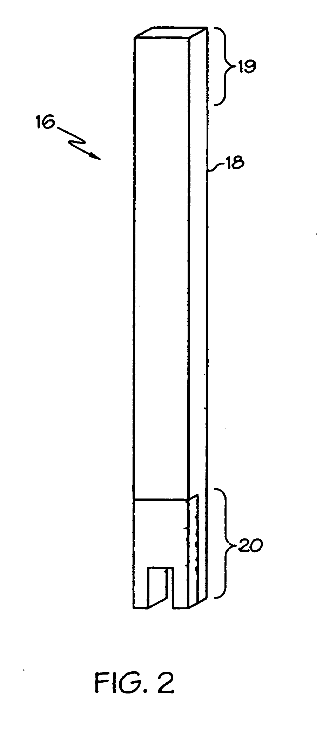

The container 5 includes a housing 10 with a cavity 12 and a lid 14. The container 5 is suitable for dispensing any type of test strip 16, for example, electrochemical and colorimetric or photometric type test strips 16 as are known in the art, where such test strips 16 find use in the determination of a wide variety of different analyte concentrations, where representative analytes include, but are not limited to, glucose, cholesterol, lactate, alcohol, and the like. In many embodiments, the subject test strips 16 are used to determine the glucose concentration in a physiological sample, e.g., interstitial fluid, blood, blood fractions, constituents thereof, and the like. In further describing the embodiments of the present disclosure, a conventional test strip 16 is described with reference made to FIG. 2 by way of example and not limitation.

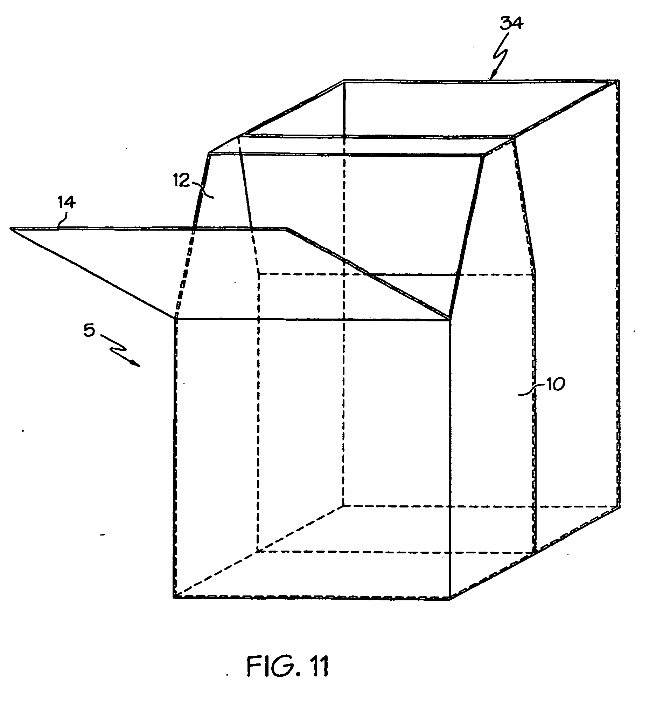

The illustrated test strip 16 shown by FIG. 2 is generally made up of at least the following components: a support element 18, and a reagent portion 20 for receiving a sample. The reagent portion 20. in one embodiment, can comprise a reagent composition that typically includes one or more members of an analyte oxidation signal producing system and a support element. The test strip 16 is typically configured and adapted to be received in an automated test strip reader/meter 34, as described below in reference to FIG. 11, for automatically determining the concentration of an analyte.

As shown, the reagent portion 20 is attached to the support element 18, in which the support element may be of a material (or material layers) that is sufficiently rigid to be inserted into the meter 34 without undue bending or kinking. In one embodiment, the support element 18 can be made of material(s) such as polyolefins, e.g., polyethylene or polypropylene, polystyrene or polyesters, and combinations thereof where in embodiments having a support element 18 formed from layers, such materials in support element 18 may be the same or different. Consequently, the length of the support element 18 typically dictates or corresponds to the length of the test strip l 6.

Regardless of whether or not the length of the support elements 18 dictates or corresponds to the length of the test strip 16, the length of the test strip 16 generally ranges from about 3 mm to about 1000 mm, usually from about 10 mm to about 100 mm and more usually from about 20 mm to about 60 mm.

As described above, the support element 18 is usually configured to enable the test strip 16 to be inserted into a test strip meter 34 (FIG. 11). As such, the support element 18, and thus the test strip 16, are typically in the form of a substantially rectangular or square-like strip, where the dimensions of the support element vary according to a variety of factors, as will be apparent to those of skill in the art, and may be the same or different.

Examples of such test strips suitable for use with the present disclosure include those described in U.S. application Ser. Nos. 09/333,793; 09/497,304; 09/497,269; 09/736,788 and 09/746,116.

Referring again to FIG. 1, the housing 10 and lid 14 may be formed of one integrated assembly. However, the housing 10 may be made up of two separate and separable assemblies: a lid 14 and a housing 10. In other words, the lid 14 and the housing 10 are not attached together. Either configuration advantageously enables substantially air and moisture tight seals to be created and maintained between the lid 14 and the housing 10.

In one container, the container 5 has a housing 10 and lid 14 comprising a rigid material that will retain its shape and form without cracking or breaking. The housing 10 and lid 14 may be manufactured from a variety of materials. In addition, where the housing 10 and lid 14 may be manufactured from the same or different materials. However, such materials will not interfere with the reagent portion 20 of the test strip 16 retained therein. Examples of such materials include, but are not limited to, plastics such as polytetrafluoroethylene, polypropylene, polyethylene, polystyrene, polycarbonate, and blends thereof. Materials may also include metals such as stainless steel, aluminum and alloys thereof, siliceous materials, and the like.

The housing 10 and lid 14 are alignable in a close configuration, such that the housing 10 and lid 14 form a substantially air and moisture tight seal when in a closed configuration. By substantially air and moisture tight seal is meant that the housing 10 and lid 14 are capable of preventing substantial air and moisture from entering the cavity 12 when the housing 10 and lid 14 are in a closed configuration.

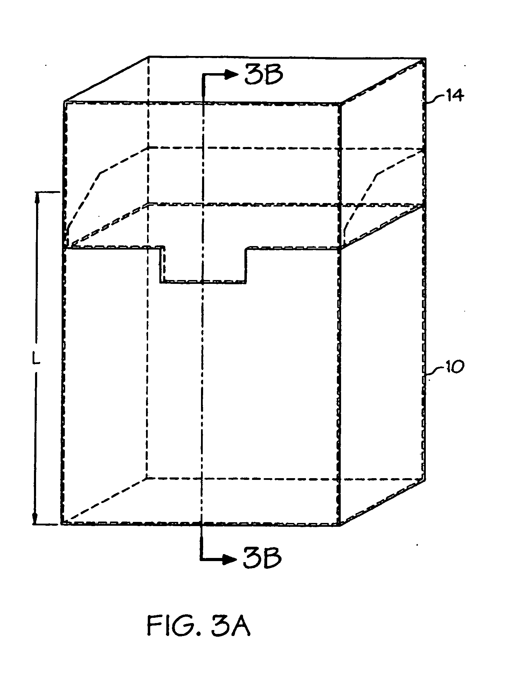

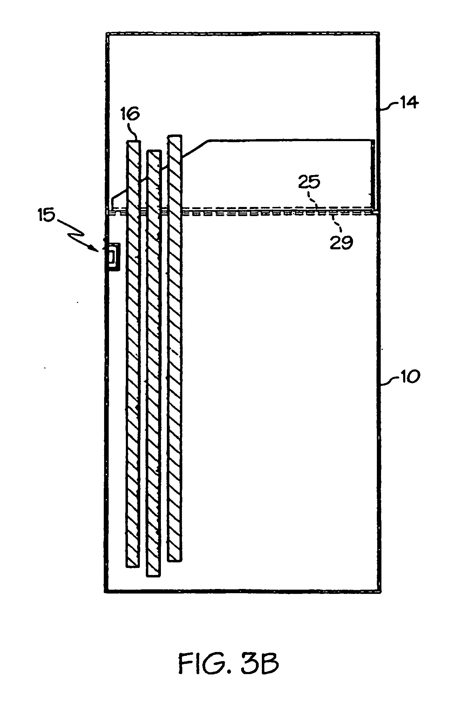

With reference to FIGS. 3A-D, in order to accomplish the substantially moisture and air free environment, the housing 10 includes an attachment means 15, where such attachment means aligns and mates, i.e., attaches, the housing 10 and lid 14 together to form a seal that is substantially air and moisture tight. Representative attachment means include, but are not limited to, at least one of: a snap fit mechanism, a frictional engagement, a lid seating mechanism, an O-ring gasket, with each of those will now be described in more detail. See FIGS. 3B-D.

Referring to Fig. 3B, in order to effect and maintain the substantially air and moisture tight seal, the lid 14 (or optionally the housing 10) may include a sealing bead 25 configured such that a corresponding groove 29 of the housing 10 (or the lid 14 if the bead is positioned on the housing 10) is configured to receive and mate with the bead 25 of the lid 14 when the lid 14 and the housing 10 are in a closed position. The bead 25 is typically tapered to enable the edges of the housing 10 (or the lid 14 if the bead 25 is positioned on the base) to fit snuggly between the bead 25 and the lid 14.

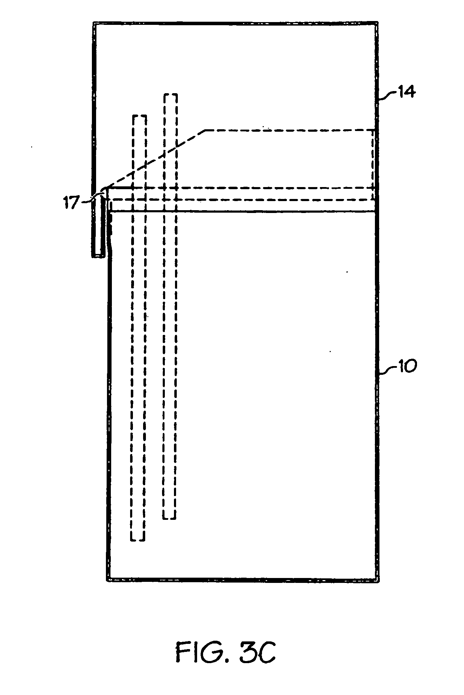

Referring again to FIG. 3C, an O-ring gasket 17 may be employed to form an intimate contact between the housing 10 and lid 14. For example, an O-ring 17 may be positioned on the housing 10 or the lid 14 to form an intimate contact when the lid 14 and housing 10 are in a closed position.

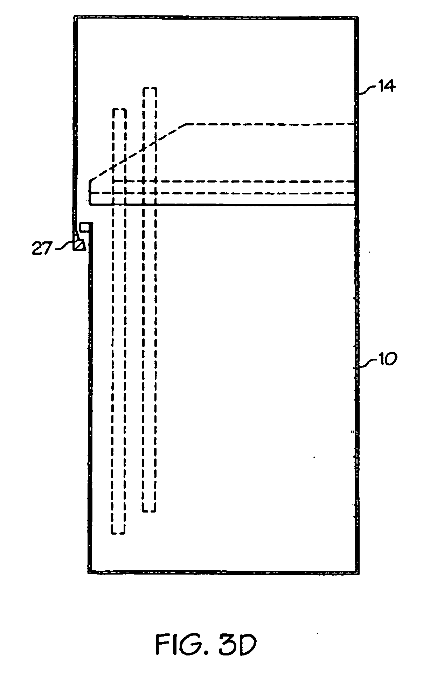

Referring again to FIG. 3D, a snap fit mechanism 27 may also be employed. By snap fit mechanism 27 is meant any suitable "built in" or integral latching mechanism for attaching one part to another. A snap fit mechanism 27 is different from loose or chemical attachment methods in that it requires no additional pieces, materials or tools to carry out the attaching function.

As noted above, one or more, sometimes two or three or more of the above described attachment means 15 may be used to create a seal between the lid 14 and the housing 10, where such a closure enables a substantially air and moisture tight seal.

The subject device may further include moisture absorbent reagents or components such as desiccant material, silica gel and the like, where such material is capable of absorbing moisture from the environment surrounding the stored test strips. Such absorbent reagents or components may be retained in one or more compartments positioned inside the housing 10 and/or lid 14.

With reference to Fig. 1, the size and shape of the housing 10 will necessarily vary depending on a variety of factors, where such factors include, but are not limited to, the type and number of test strips 16 retained therein, and the like. Accordingly, the shape of the housing 10 may take any of a variety of shapes. For example, the housing 10 may be substantially rectangular, substantially square, substantially cylindrical, substantially round, substantially circular, substantially elliptical or substantially oval shape. Alternatively, the shape may be more complex such as a substantially irregular shape or the like. The four corners of the container of the housing 10 are typically rounded or beveled to avoid any snagging or injury by the user.

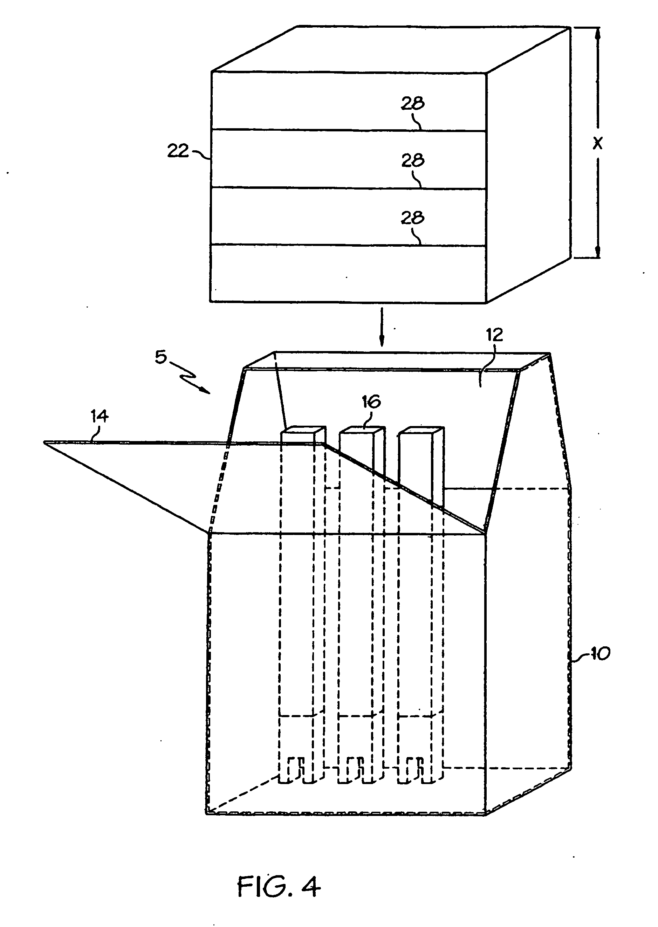

Reference now is made to FIG. 4, in which a container 5 is provided with a compressible insert 22 . The compressible insert 22 may comprise a compressible material that expands upon release of external pressure. In one embodiment, the compressible insert 22 comprises an open-cell foam. In another embodiment, the compressible insert 22 comprises a gel-filled pillow. The compressible insert 22 may also comprise any material that may be easily compressed and then expand upon release of external pressure such as rubber, foam, synthetic rubber, and compressible plastic.

The compressible insert 22 is used to protect and retain the test strips 16 within the cavity 12 of the housing 10. In one embodiment, ridges 28 are perpendicularly disposed along the longitudinal axis X of the compressible insert 22. In one embodiment, the ridges 28 are spaced no more than about 10 mm apart from one another. In yet another embodiment, the ridges 28 are spaced no more than about 5 mm apart from one another. In another embodiment, the ridges 28 may be spaced any distance from one another so long as the test strips 16 are suitably retained when held thereby, as described below.

It is to be appreciated that the compressible insert 22 can be manually placed and removed to and from the cavity 12. Such a feature permits the easy loading of additional test strips as desired. When placed in the cavity, the compressible insert 22 is frictionally retained therein due to its expansion and conform to the cavity 12. The compressible insert 22 may be inserted into the cavity 12 before insertion of a plurality of test strips 16. The test strips 16 may be inserted in the front of the compressible insert 22 by manual insertion. Alternatively, in another embodiment, the test strips 16 may be placed within the cavity 12, and then the compressible insert 22 may be insert behind the test strips 16. Upon insertion the compressible insert 22 as mentioned will expand to releasably retain the test strips 16 in the front of the cavity 12. In other embodiments, the compressible insert 22 may be attached to the inside of the cavity 12 by adhesive, or mechanical mounting.

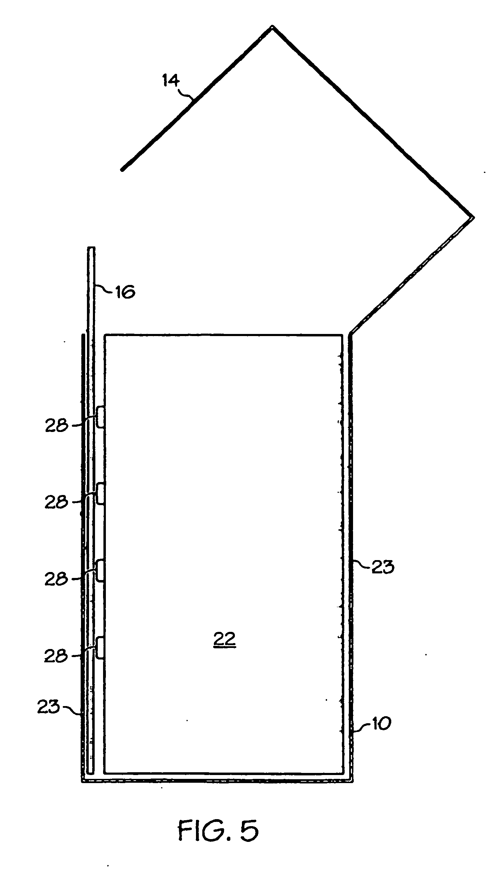

FIG. 5 is a cross-sectional view of the compressible insert 22 inserted in the housing 10 for storing a test strip 16 with a series of the ridges 28 in accordance with one embodiment. In this illustrated embodiment, the ridges 28 operate to form a barrier against air and water ingress into the cavity 12. The compressible insert 22 expands inside the cavity 12 which presses the ridges 28 against the interior surfaces or inner walls 23 of the housing 10. The ridges 28 deform to accommodate the test strips within the cavity 12, and form a seal against air and water ingress. The ridges 28 comprise a raised portion of the compressible insert 22. In one embodiment, each ridge 28 is typically about 0.5 mm to about 2 mm tall, relative to the compressible insert 22, and each ridge 28 is typically about 30 to about 50 mm long, spanning the entire length of the compressible insert 22. In one embodiment, the ridges 28 comprise half-moon extruded along perpendicular line. In another embodiment, the ridges 28 comprise a triangular shape extruded along perpendicular line.

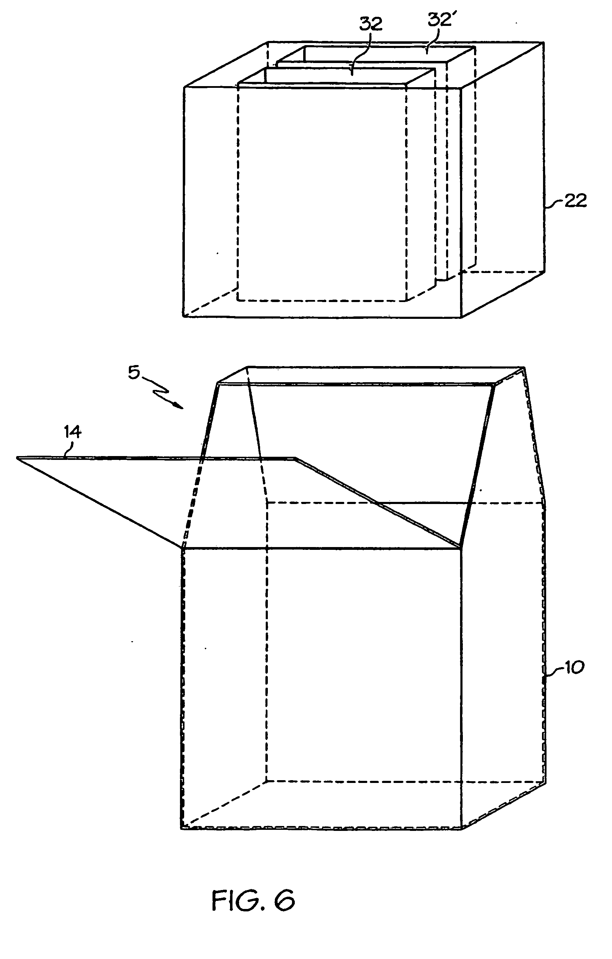

Another embodiment of the compressible insert 22 useable in the container 5 is illustrated in FIG. 6. In one embodiment, the compressible insert 22 contains at least one slot 32 longitudinally disposed along the compressible insert 22. In one embodiment, the compressible insert 22 comprises only one slot 32 sized to hold one to ten test strips 16. In another embodiment, the compressible insert 22 comprises only one slot 32 sized to hold approximately five to twenty test strips 16. In still other embodiment, an additional slot(s) 32' may be provided adjacent to slot 32 in a similar manner and for a similar purpose.

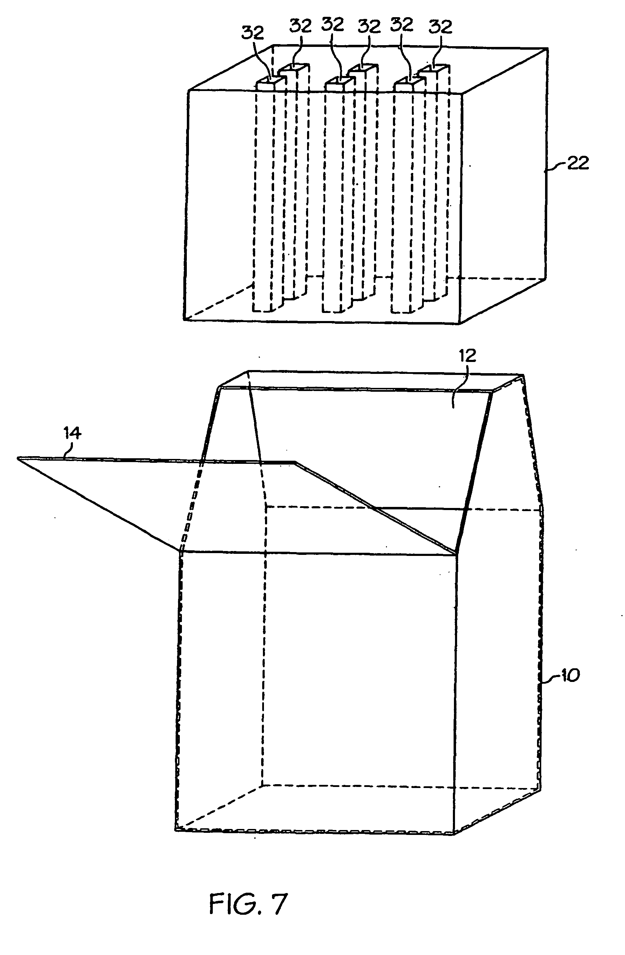

Still another compressible insert 22 useable with the container 5 is shown in FIG. 7. In this illustrated embodiment, the compressible insert 22 comprises approximately one to six slots 32 substantially parallel to one another, each sized to retain approximately one to five test strips 16. Another compressible insert 22 comprises approximately five to ten slots 32, each sized to retain one test strip 16.

The compressible insert 22 may be shaped to resemble the housing 10. The compressible insert 22 may comprise the shape of substantially a cube, rectangular prism, elliptical shape. Alternatively, it may be shaped in an irregular fashion other than that which has been disclosed.

Referring to Fig. 5, the compressible insert 22 may be oriented in the cavity 12 in any fashion that would provide substantial contact between the compressible insert 22 and the inner wall 23 of the housing 10. In one embodiment, the compressible insert 22 aligns substantially with the inner walls 23 of the housing 10, thereby filling the cavity 12. In another embodiment, the compressible insert 22 aligns with the inner walls 23 of the housing 10 only below the opening 21 (FIG. 1). In such an embodiment, upon insertion of the compressible insert 22 in different orientations, the compressible insert 22 will expand to substantially conform to the shape of the cavity 12.

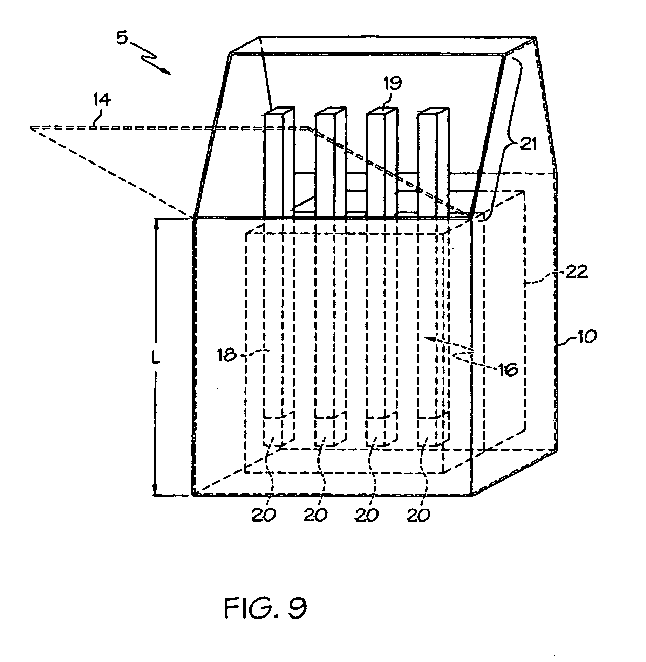

Referring to Fig. 9, the size of the housing 10 may also vary depending on a variety of factors such as the type and number of test strips 16 retained therein. In certain embodiments, the housing 10 is configured such that the plurality of test strips is retained in the cavity 12 of housing 10. In the illustrated embodiment, the housing 10 and lid 14 are sized and arranged such that a handling portion 19 of the support element I of each test strips 16 retained in the housing 10. The lid 14 is used to close this opening 21. In this manner, when the lid 14 is opened, the handling portion 19 of each retained test strip 16 that extends beyond the housing 10 enables an individual to easily grasp a single test strip 16 from the opening 21, while avoiding many of the problems associated with prior art devices. For example, height L of the housing 10 is typically about two thirds the length of each test strip 16 (i.e., about one-third of each test strip protrudes above the distal edge of the housing 10, where the height of the housing 10 may be as little as one half or less the length of each test strip 16.

The size of the compressible insert 22 may also vary depending on a variety of factors such as the type and number of test strips 16 retained therein, or the size of the cavity 12 into which the insert is placed. In one embodiment, the compressible insert 22 has an about 40% larger volume than the cavity 12. In another embodiment, the compressible insert 22 has an about 25% larger volume than the cavity 12. The compressible insert 22 is compressible to fit within a smaller volume than originally provided. In one embodiment, the compressible insert 22 may be compressed to about 50% of its original volume. In another embodiment, the compressible insert 22 may be compressed to about 70% of its original volume. However, the compressible insert 22 may have a wide range of volumes in relation to the size of the cavity 12.

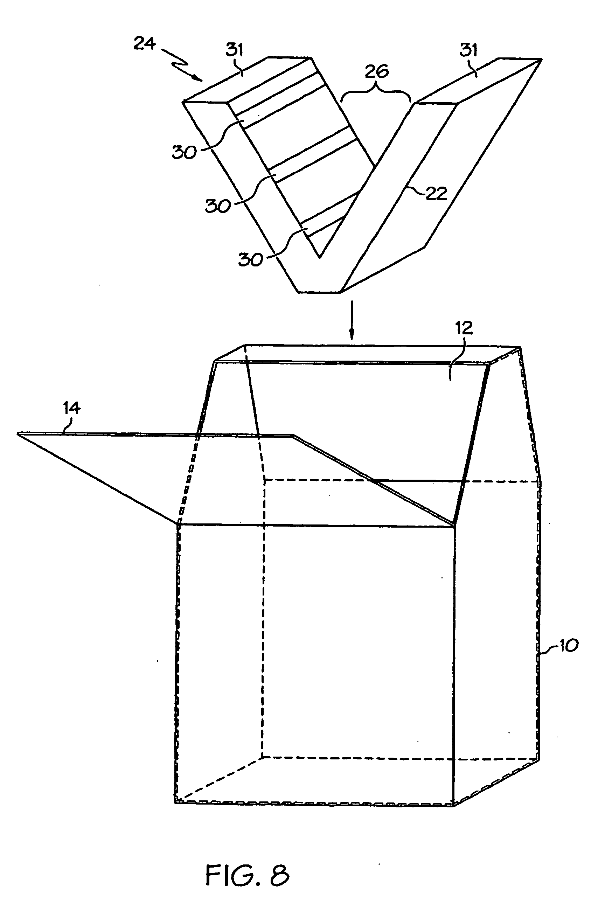

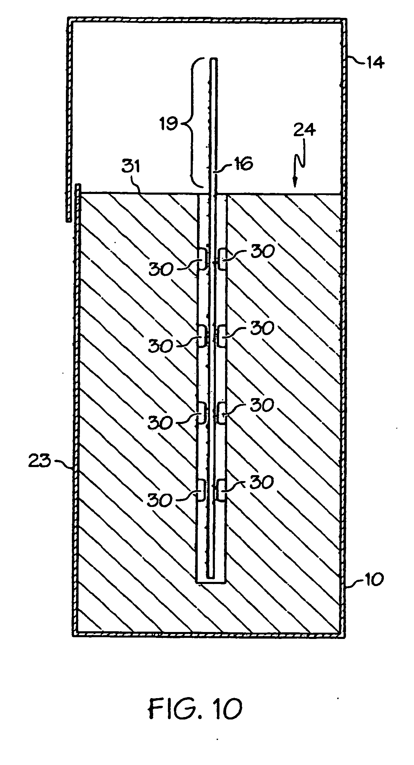

The dimensions of the compressible insert 22 may also vary depending on a variety of factors such as the type and number of test strips retained in the housing 10, or the size of the cavity 12 placed into. In one embodiment, the length of the compressible insert 22 is more then twice the length of the test strip 16, where the compressible insert 22 is folded to form a folded insert 24 (FIG. 8) so the handling portion 19 (FIG. 10) extends beyond the distal or unfolded distal edges 31 of the folded insert 24, so to enable an individual to easily grasp a single test strip 16 while avoiding many of the problems associated with prior art devices. For example, the length of the folded insert 24 may be less than about twice of the length of a test strip (i.e., about one-third of each test strip protrudes above the distal edges 31 of the folded insert 24).

With reference to FIG. 8, as mentioned above, the compressible insert 22 may be folded to form the folded insert 24. The compressible insert 22 may be folded in any method to define a storage chamber 26 between the two folded halves of the folded insert 24. In one embodiment, the compressible insert 22 is longitudinally folded (i.e., along its longest length) to define the storage chamber 26. Alternatively, the compressible insert 22 can be latitudinally folded (i.e., along its shorter length) to also define the storage chamber 26. In one embodiment, the compressible insert 22 may be folded along its midpoint along its longer length to form the folded insert 24, such as depicted by FIG. 8. In another embodiment, the compressible insert 22 may be folded at a point different than the midpoint of the compressible insert 22. In another embodiment, the folded insert 24 may comprise a series of folds that define at least one storage chamber 26.

Storage chamber 26 of the folded insert 24 may be configured to retain from about 1 to about 25 test strips at one time, usually about I to about 10, however the storage chamber 26 may be configured to retain more or fewer test strips as desired. The storage chamber 26 may hold less than about ten test strips 16. In another container, the storage chamber 26 holds more than about ten test strips 16, but not more than about twenty test strips 16. In another container, the storage chamber 26 may hold from about ten to fifty test strips 16.

The reagent portion 20 of the test strips 16 may be enclosed in the storage chamber 26 of the folded insert 24 as depicted by FIG. 9. In one container, the folded insert 24 completely surrounds the reagent portion 20 of the test strips 16 to protect them from environmental degradation. In another embodiment, as best shown by FIG. 10, cooperating ridges 30 of the compressible insert 22 cooperate to form a series of successive barriers upon folding of the compressible insert 22. The cooperating ridges 30 align upon folding of the compressible insert 22 to form a seal operable to prevent environmental degradation. The cooperating ridges 30 comprise a raised portion of the compressible insert 22. In one embodiment, each cooperating ridge 30 is typically about 0.5 mm to 2 mm tall, relative to the compressible insert 22, and each cooperating ridge 30 is typically about 30 to about 50 mm long, spanning the entire length of the compressible insert 22. In one embodiment, the cooperating ridges 30 comprise half moon extruded substantially parallel line to the distal edge 31. In another embodiment, the cooperating ridges 30 comprise a substantially triangular shape extruded substantially parallel line to the distal edge 31. In alternative embodiments, the cooperating ridges 30 may form any shape operable to form a seal around the test strips 16.

The compressible insert 22 may have a coating to repel dirt, water, and lint from the compressible insert 22. The coating may comprise any material that repels dirt, water, and lint. In another embodiment, the coating may comprise a finish texture that repels dirt, water, and lint. In another embodiment, the coating is integral with the compressible insert 22.

FIG. 11 is a perspective view of a container 5 in combination with a test strip meter 34. The housing 10 may be connected to a test strip meter 34 configured to analyze test strips 16, and display results to the user. The housing 10 may be integrated into the main body of a test strip meter 34 (FIG. 11). Such test strip meters 34 are well known in the art, and operable to analyze and process sample contained within the plurality of test strips.

The container may have other shapes, such as circular, oval, trapezoidal; and the test strips may be oriented in a different fashion.

Thus the scope of the invention is limited only by the appended claims.

## Claims

1. A container (5) for storing a plurality of test strips (16), each test strip having a reagent portion (20) and a handling portion (19), the container (5) comprising:
a housing (10) defining a cavity (12);
a lid (14) hingedly connected to the housing (10) to close the cavity (12); and
a compressible insert (22) mounted in the cavity (12), whereby the compressible insert (22, 24) is expandable to retain the plurality of test strips (16) in the cavity (12), **characterized in that** the insert further comprises ridges (28) which operate to form a barrier against air and water ingress into the cavity (12), whereby said ridges (28) comprises a raised portion of said compressible insert (22).

2. The container of claim 1, wherein the compressible insert (22, 24) further comprises at least one ridge (28, 30).

3. The container of claim 1, wherein the compressible insert (22, 24) comprises open-cell foam.

4. The container of claim 1, wherein the compressible insert (22, 24) further comprises a coating.

5. The container for claim 1, further comprising a desiccant.

6. The container of claim 1, wherein the housing (10) and the compressible insert (22, 24) are proportioned to allow the handling portion (19) of the plurality of test strips (16) to protrude from the housing (10), wherein a user may remove at least one test strip (16).

7. The container of claim 1, wherein the compressible insert (22, 24) comprises a gel-filled pillow.

8. The container of claim 1, wherein the compressible insert (22) is a folded insert (24), wherein a folded portion defines a storage chamber (26) to retain the plurality of test strips (16).

9. The container of claim 1, wherein the compressible insert (24) is folded to encompass a reagent portion (20) of the plurality of test strips (16).

10. A method for storing a plurality of test strips (16) comprising:
providing a container (5) for storing a plurality of test strips (16), each test strip (16) having a reagent portion (20) and a handling portion (19), the container (5) comprising:
a housing (10) defining a cavity (12),
a lid (14) hingedly connected to the housing (10) to close the cavity (12), and
a compressible insert (22, 24) removably mounted in the cavity (12), the compressible insert (22, 24) is expandable to retain the plurality of test strips (16) in the cavity (12);
providing a plurality of test strips (16) in the cavity (12); and
**characterized by**
storing the plurality of test strips (16) in the cavity (12), wherein the compressible insert (22, 24) expands to releasably retain the plurality of test strips (16) and protect the plurality of test strips (16) from environmental degradation and the compressible insert further comprises ridges (28) which operate to form a barrier against air and water ingress into the cavity (12), whereby said ridges (28) comprise a raised portion of said compressible insert (22).

11. The method of claim 10, further comprising folding the compressible insert (22, 24) to encompass a majority of the surface area of the plurality of test strips (16).

12. The method of claim 10, wherein the housing (10) and the compressible insert (22, 24) are proportioned to allow the handling portion (19) of the plurality of test strips (16) to protrude from the housing (10), wherein a user may remove at least one test strip (16).

13. A method of manufacturing a container (10) for storing a plurality of test strips (16) comprising:
providing a housing (10) with a defined cavity (12) and a lid (14) hingedly connected to the housing (10) for closing the cavity (12); and
**characterized by**
folding and inserting a compressible insert (22, 24) into the cavity, the compressible insert (22, 24) being expandable to retain the plurality of test strips 16) in the cavity (12).-, wherein the compressible insert (22, 24) further comprises ridges (28) which operate to form a barrier against air and water ingress into the cavity (12), whereby said ridges (28) comprise a raised portion of said compressible insert (22).

## Patentansprüche

1. Behälter (5) zum Aufbewahren von mehreren Teststreifen (16), wobei jeder Teststreifen einen Reagenzabschnitt (20) und einen Handhabungsabschnitt (19) aufweist, wobei der Behälter (5) Folgendes aufweist:
ein Gehäuse (10), das einen Hohlraum (12) definiert;
einen Deckel (14), der zum Schließen des Hohlraums (12) an das Gehäuse (10) angelenkt ist; und
einen komprimierbaren Einsatz (22), der in dem Hohlraum (12) angebracht ist, wobei der komprimierbare Einsatz (22, 24) zum Halten der mehreren Teststreifen (16) in dem Hohlraum (12) ausdehnbar ist, **dadurch gekennzeichnet, dass** der Einsatz ferner Stege (28) aufweist, die zum Ausbilden einer Barriere gegen das Eindringen von Luft und Wasser in den Hohlraum (12) arbeiten, wobei die Stege (28) einen gehobenen Abschnitt des komprimierbaren Einsatzes (22) umfassen.

2. Behälter nach Anspruch 1, wobei der komprimierbare Einsatz (22, 24) mindestens einen Steg (28, 30) aufweist.

3. Behälter nach Anspruch 1, wobei der komprimierbare Einsatz (22, 24) offenzelligen Schaum aufweist.

4. Behälter nach Anspruch 1, wobei der komprimierbare Einsatz (22, 24) ferner eine Beschichtung aufweist.

5. Behälter nach Anspruch 1, ferner aufweisend ein Trockenmittel.

6. Behälter nach Anspruch 1, wobei das Gehäuse (10) und der komprimierbare Einsatz (22, 24) zum Ermöglichen dimensioniert sind, dass der Handhabungsabschnitt (19) der mehreren Teststreifen (16) aus dem Gehäuse (10) vorragt, wodurch ein Benutzer mindestens einen Teststreifen (16) entfernen kann.

7. Behälter nach Anspruch 1, wobei der komprimierbare Einsatz (22, 24) ein gelgefülltes Polster aufweist.

8. Behälter nach Anspruch 1, wobei der komprimierbare Einsatz (22) ein gefalteter Einsatz (24) ist, wobei ein gefalteter Abschnitt eine Aufbewahrungskammer (26) zum Halten der mehreren Teststreifen (16) definiert.

9. Behälter nach Anspruch 1, wobei der komprimierbare Einsatz (24) zum Einschließen eines Reagensabschnitts (20) der mehreren Teststreifen (16) gefaltet ist.

10. Verfahren zum Aufbewahren von mehreren Teststreifen (16), aufweisend:
Bereitstellen eines Behälters (5) zum Aufbewahren von mehreren Teststreifen (16), wobei jeder Teststreifen (16) einen Reagensabschnitt (20) und einen Handhabungsabschnitt (19) aufweist, wobei der Behälter (5) Folgendes aufweist:
ein Gehäuse (10), das einen Hohlraum (12) definiert;
einen Deckel (14), der zum Schließen des Hohlraums (12) an das Gehäuse (10) angelenkt ist; und
einen komprimierbaren Einsatz (22, 24), der in dem Hohlraum (12) entfernbar angebracht ist, wobei der komprimierbare Einsatz (22, 24) zum Halten der mehreren Teststreifen (16) in dem Hohlraum (12) ausdehnbar ist;
Bereitstellen von mehreren Teststreifen (16) in dem Hohlraum (12); und
**gekennzeichnet durch**
Aufbewahren der mehreren Teststreifen (16) in dem Hohlraum (12), wobei sich der komprimierbare Einsatz (22, 24) zum lösbaren Halten der mehreren Teststreifen (16) und Schützen der mehreren Teststreifen (16) vor umgebungsbedingter Schädigung ausdehnt und der komprimierbare Einsatz ferner Stege (28) aufweist, die zum Ausbilden einer Barriere gegen das Eindringen von Luft und Wasser in den Hohlraum (12) arbeiten, wobei die Stege (28) einen gehobenen Abschnitt des komprimierbaren Einsatzes (22) umfassen.

11. Verfahren nach Anspruch 10, ferner aufweisend das Falten des komprimierbaren Einsatzes (22, 24) zum Einschließen eines Großteils des Oberflächenbereichs der mehreren Teststreifen (16).

12. Verfahren nach Anspruch 10, wobei das Gehäuse (10) und der komprimierbare Einsatz (22, 24) zum Ermöglichen dimensioniert sind, dass der Handhabungsabschnitt (19) der mehreren Teststreifen (16) aus dem Gehäuse (10) vorragt, wodurch ein Benutzer mindestens einen Teststreifen (16) entnehmen kann.

13. Verfahren zum Herstellen eines Behälters (10) zum Aufbewahren von mehreren Teststreifen (16), aufweisend:
Bereitstellen eines Gehäuses (10) mit einem definierten Hohlraum (12) und einem Deckel (14), der zum Schließen des Hohlraums (12) an das Gehäuse (10) angelenkt ist; und
**gekennzeichnet durch**
Falten und Einsetzen eines komprimierbaren Einsatzes (22, 24) in den Hohlraum, wobei der komprimierbare Einsatz (22, 24) zum Halten der mehreren Teststreifen (16) in dem Hohlraum (12) ausdehnbar ist, wobei der komprimierbare Einsatz (22, 24) ferner Stege (28) aufweist, die zum Ausbilden einer Barriere gegen das Eindringen von Luft und Wasser in den Hohlraum (12) arbeiten, wobei die Stege (28) einen gehobenen Abschnitt des komprimierbaren Einsatzes (22) umfassen.

## Revendications

1. Boîtier (5) pour stocker une pluralité de bandelettes de test (16), chaque bandelette de test ayant une partie de réactif (20) et une partie de manipulation (19), le boîtier (5) comprenant :
un logement (10) définissant une cavité (12) ;
un couvercle (14) raccordé par charnière au logement (10) pour fermer la cavité (12) ; et
un insert compressible (22) monté dans la cavité (12), ce par quoi l'insert compressible (22, 24) est expansible pour maintenir la pluralité de bandelettes de test (16) dans la cavité (12), **caractérisé en ce que** l'insert comprend en outre des nervures (28) qui fonctionnent de façon à former une barrière contre une entrée d'air et d'eau à l'intérieur de la cavité (12), ce par quoi lesdites nervures (28) comprennent une partie surélevée dudit insert compressible (22).

2. Boîtier selon la revendication 1, dans lequel l'insert compressible (22, 24) comprend en outre au moins une nervure (28, 30).

3. Boîtier selon la revendication 1, dans lequel l'insert compressible (22, 24) comprend une mousse à alvéoles ouvertes.

4. Boîtier selon la revendication 1, dans lequel l'insert compressible (22, 24) comprend en outre un revêtement.

5. Boîtier selon la revendication 1, comprenant en outre un dessiccant.

6. Boîtier selon la revendication 1, dans lequel le logement (10) et l'insert compressible (22, 24) sont proportionnés de façon à permettre que la partie de manipulation (19) de la pluralité de bandelettes de test (16) fasse saillie du logement (10), dans lequel un utilisateur peut prélever au moins une bandelette de test (16).

7. Boîtier selon la revendication 1, dans lequel l'insert compressible (22, 24) comprend un coussin rempli de gel.

8. Boîtier selon la revendication 1, dans lequel l'insert compressible (22) est un insert plié (24), dans lequel une partie pliée définit une chambre de stockage (26) pour maintenir la pluralité de bandelettes de test (16).

9. Boîtier selon la revendication 1, dans lequel l'insert compressible (24) est plié de façon à couvrir une partie de réactif (20) de la pluralité de bandelettes de test (16).

10. Procédé de stockage d'une pluralité de bandelettes de test (16) comprenant :
la fourniture d'un boîtier (5) pour stocker une pluralité de bandelettes de test (16), chaque bandelette de test ayant une partie de réactif (20) et une partie de manipulation (19), le boîtier (5) comprenant :
un logement (10) définissant une cavité (12),
un couvercle (14) raccordé par charnière au logement (10) pour fermer la cavité (12), et
un insert compressible (22, 24) monté de façon amovible dans la cavité (12), l'insert compressible (22, 24) étant est expansible pour maintenir la pluralité de bandelettes de test (16) dans la cavité (12) ;
la prévision d'une pluralité de bandelettes de test (16) dans la cavité (12) ; et
**caractérisé par**
le stockage de la pluralité de bandelettes de test (16) dans la cavité (12), dans lequel l'insert compressible (22, 24) est expansé pour maintenir de manière amovible la pluralité de bandelettes de test (16) et protéger la pluralité de bandelettes de test (16) d'une dégradation environnementale et l'insert compressible comprend en outre des nervures (28) qui fonctionnent de façon à former une barrière contre une entrée d'air et d'eau à l'intérieur de la cavité (12), ce par quoi lesdites nervures (28) comprennent une partie surélevée dudit insert compressible (22).

11. Procédé selon la revendication 10, comprenant en outre le pliage de l'insert compressible (22, 24) pour couvrir une majorité de la superficie de la pluralité de bandelettes de test (16).

12. Procédé selon la revendication 10, dans lequel le logement (10) et l'insert compressible (22, 24) sont proportionnés de façon à permettre que la partie de manipulation (19) de la pluralité de bandelettes de test (16) fasse saillie du logement (10), dans lequel un utilisateur peut prélever au moins une bandelette de test (16).

13. Procédé de fabrication d'un boîtier (10) pour stocker une pluralité de bandelettes de test (16) comprenant :
la prévision d'un logement (10) avec une cavité (12) définie et un couvercle (14) raccordé par charnière au logement (10) pour fermer la cavité (12) ; et
**caractérisé par**
le pliage et l'insertion d'un insert compressible (22, 24) à l'intérieur de la cavité, l'insert compressible (22, 24) étant expansible pour maintenir la pluralité de bandelettes de test (16) dans la cavité (12), dans lequel l'insert compressible (22, 24) comprend en outre des nervures (28) qui fonctionnent de façon à former une barrière contre une entrée d'air et d'eau à l'intérieur de la cavité (12), ce par quoi lesdites nervures (28) comprennent une partie surélevée dudit insert compressible (22).
